# EUROPÄISCHE PATENTSCHRIFT

(11) **EP 1 001 929 B1**
(45) Veröffentlichungstag und Bekanntmachung des Hinweises auf die Patenterteilung: **26.03.2003**
(21) Anmeldenummer: 98943740.5
(22) Anmeldetag: 18.07.1998
(51) Int. Cl.: C07C 253/30, C07C 255/51, C07C 255/57

(54) **VERFAHREN ZUR HERSTELLUNG VON 3-CYANO-2,4-DIHALOGEN-5-FLUOR-BENZOESÄUREN**
METHOD FOR PREPARING 3-CYANO-2,4-DIHALOGEN-5-FLUOR-BENZOIC ACID
PROCEDE POUR LA PREPARATION D'ACIDE 3-CYANO-2, 4-DIAHALOGENE-5-FLUOR-BENZOIQUE

(30) Priorität: 01.08.1997 DE 19733243
(43) Veröffentlichungstag der Anmeldung: 24.05.2000
(73) Patentinhaber: Bayer Aktiengesellschaft, 51368 Leverkusen (DE)
(72) Erfinder: HALLENBACH, Werner, D-40789 Monheim (DE); MARHOLD, Albrecht, D-51373 Leverkusen (DE)
(86) Internationale Anmeldenummer: EP9804468
(87) Internationale Veröffentlichungsnummer: WO99006360

(56) Entgegenhaltungen:
- EP-A- 0 307 897
- WO-A-97/31001
- WO-A-98/26768
- DE-A- 3 702 393

## Beschreibung

Die vorliegende Erfindung betrifft ein Verfahren zur Herstellung von 3-Cyano-2,4-dihalogen-5-fluor-benzoesäuren, Zwischenprodukte zur Durchführung des Verfahrens und Verfahren zur Herstellung dieser Zwischenprodukte.

3-Cyano-2,4-dichlor-5-fluor-benzoesäure ist bekannt aus DE-A-3 702 393. Sie wird hergestellt aus 3-Amino-2,4-dichlor-5-fluor-benzoesäure durch Diazotierung und Umsetzung des Diazoniumsalzes mit Cyanidsalzen. Dieses Verfahren ist vor allem bei der Durchführung in größerem Maßstab ungünstig.

Gegenstand der vorliegenden Erfindung ist
1. Verfahren zur Herstellung von 3-Cyano-2,4-dihalogen-5-fluorbenzoesäuren der Formel (I) 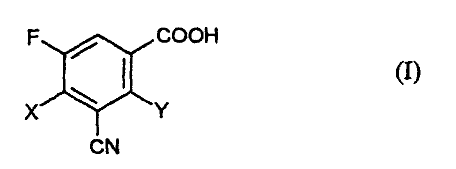 in welcher
   - X und Y: unabhängig voneinander für Halogen stehen,
   durch hydrolytische Spaltung von
   a) 3-Cyano-2,4-dihalogen-5-fluorbenzoesäureamiden der Formel (II) 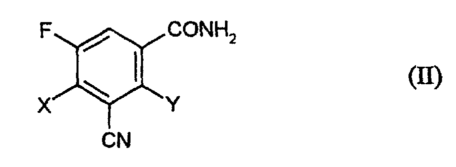 in welcher
      - X und Y: unabhängig voneinander für Halogen stehen,
      oder
   b) 1,3-Dicyano-2,4-dihalogen-5-fluorbenzolen der Formel (III) 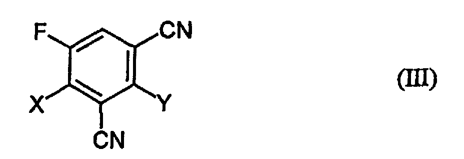 in welcher
      - X und Y: unabhängig voneinander für Halogen stehen,
      oder
   c) 3-Cyano-2,4-dihalogen-5-fluorbenzoesäureestern der Formel (IV), ausgenommen 3-Cyano-2,4,5-trifluorbenzoesäuremethylester, 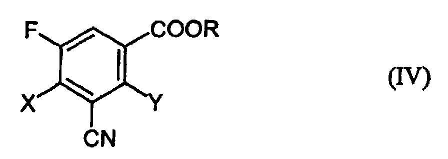 in welcher
      - X und Y: unabhängig voneinander für Halogen stehen und
      - R: für C₁₋₄-Alkyl steht, das gegebenenfalls substituiert sein kann.
2. Die neuen Verbindungen der Formeln (II) 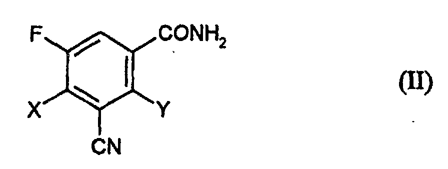 und (IV) 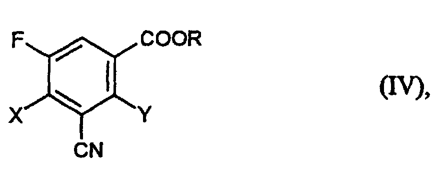 in welcher
   - X und Y: unabhängig voneinander für Halogen stehen und
   - R: für C₁₋₄-Alkyl steht, das gegebenenfalls substituiert sein kann,
   ausgenommen 3-Cyano-2,4,5-trifluor-benzoesäuremethylester.
3. Verfahren zur Herstellung der 3-Cyano-2,4-dihalogen-5-fluor-benzoesäureamide der Formel (II) 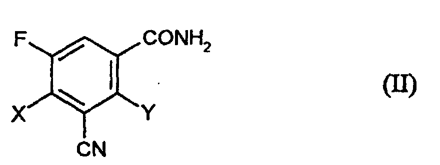 bzw. der Ester der Formel (IV) 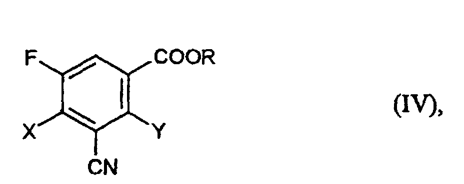 das dadurch gekennzeichnet ist, daß man 1,3-Dicyano-2,4-dihalogen-5-fluorbenzole der Formel (III) 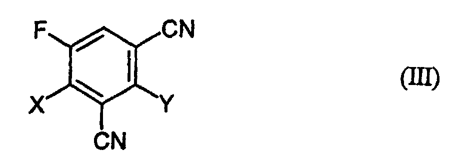 in welcher
   - X und Y: die oben angegebene Bedeutung haben,
   in Gegenwart von Wasser oder in Gegenwart von Alkoholen hydrolysiert.
4. Die neuen Verbindungen der Formel (III) 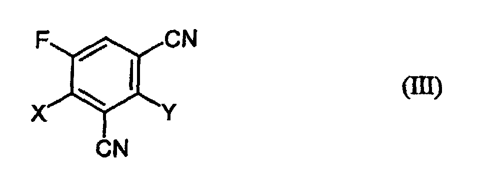 in welcher
   - X und Y: für ungleiche Reste aus der Gruppe Fluor oder Chlor stehen oder beide Reste für Chlor stehen.
5. Verfahren zur Herstellung der Verbindungen der Formel (III) 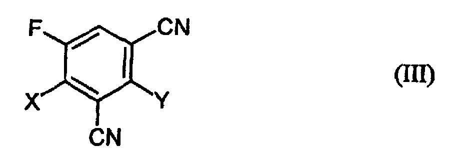 in welcher
   - X und Y: für ungleiche Reste aus der Gruppe Fluor oder Chlor stehen oder beide Reste für Chlor stehen,
   das dadurch gekennzeichnet ist, daß man 1,2,4-Trifluor-3,5-dicyanbenzol (2,4,5-Trifluor-isophthalodinitril) mit einem Metallchlorid umsetzt.

1,2,4-Trifluor-3,5-dicyanbenzol und seine Herstellung ist aus EP-A-307 897 bekannt.

In den obigen Formeln stehen X und Y bevorzugt für Fluor oder Chlor. Besonders bevorzugt stehen sie in den Verbindungen der Formeln (II) und (IV) für gleiche Reste Fluor oder Chlor.

Bei den Verbindungen der Formel (III) ist 2,4-Dichlor-5-fluorisophthalodinitril besonders bevorzugt.

R steht bevorzugt für Methyl, Ethyl, Propyl oder Benzyl.

Setzt man als Ausgangsprodukt bei Verfahren a) zur Herstellung von 3-Cyano-2,4,5-trifluorbenzoesäure das 3-Cyano-2,4,5-trifluor-benzamid ein, läßt sich die Umsetzung durch folgendes Formelschema verdeutlichen:

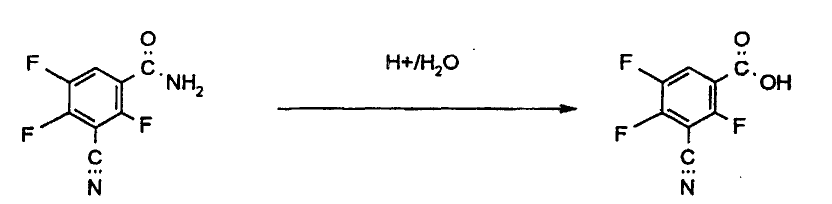

Die als Ausgangsprodukte verwendeten Amide der Formel (II) sind neu. Ihre Herstellung ist weiter unten beschrieben.

Die Hydrolyse erfolgt in Gegenwart von Säuren und Wasser. Als Säuren können organische und anorganische starke Säuren verwendet werden. Als solche seien genannt HCl, HBr, Schwefelsäure, Methansulfonsäure, Trifluormethansulfonsäure, Benzolsulfonsäure, Toluolsulfonsäure und stark saure Ionentauscher in Gegenwart von Wasser.

Als Lösungsmittel kann die als Reagenz verwendete Säure im Überschuß oder ein organisches Lösungsmittel verwendet werden. Als organische Lösungsmittel kommen in Betracht Säuren wie Ameisensäure, Essigsäure, Propionsäure, Ether wie Dimethoxyethan, Dioxan, Ketone wie Aceton, Butanon.

Die Reaktionskomponenten können in beliebiger Reihenfolge zusammengegeben werden. Anschließend wird auf die benötigte Temperatur erhitzt.

Die Reaktionstemperatur liegt im Bereich von 0 bis 200°C, bevorzugt 20 bis 150°C.

Die Reaktion kann drucklos oder unter einem Druck von 0 bis 50 bar, bevorzugt 0 bis 6 bar, durchgeführt werden.

Die Produkte werden aus der Reaktionsmischung eventuell nach Verdünnung mit Wasser abfiltriert. Wird die Säure im großem Überschuß oder ein Lösungsmittel verwendet, so kann es vorteilhaft sein abzudestillieren und das Produkt durch Extraktion zu isolieren.

Setzt man als Ausgansprodukt zur Herstellung von 4-Chlor-2,5-difluor-3-cyanbenzoesäure gemäß Verfahren 1b) 4-Chlor-2,5-difluor-isophthalodinitril ein, so läßt sich die Umsetzung durch folgendes Formelschema verdeutlichen: 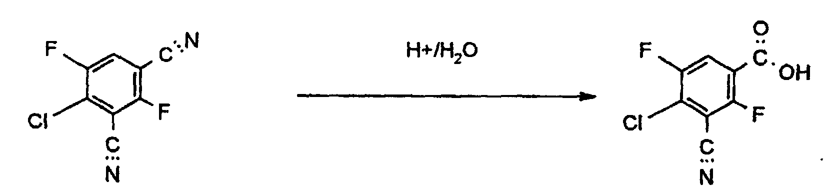 2,4,5-Trifluor-isophthalodinitril ist literaturbekannt (EP-A-307 897). 2,4-Dichlor-5-fluor-isophthalodinitril ist neu. Seine Herstellung ist weiter unten beschrieben.

Die Hydrolyse erfolgt mit Säuren in Gegenwart von Wasser. Als Säuren können organische und anorganische starke Säuren verwendet werden. Als solche seien genannt HCl, HBr, Schwefelsäure, Methansulfonsäure, Trifluormethansulfonsäure, Benzolsulfonsäure, Toluolsulfonsäure und stark saure Ionentauscher in Gegenwart von Wasser.

Als Lösungsmittel kann die als Reagenz verwendete Säure im Überschuß oder ein organisches Lösungsmittel verwendet werden. Als organische Lösungsmittel kommen in Betracht Säuren wie Ameisensäure, Essigsäure, Propionsäure, Ether wie Dimethoxyethan, Dioxan, Ketone wie Aceton, Butanon.

Die Reaktionskomponenten können in beliebiger Reihenfolge zugegeben werden. Anschließend wird auf die benötigte Temperatur erhitzt.

Die Reaktionstemperatur liegt im Bereich von 0 bis 200°C, bevorzugt 20 bis 150°C.

Die Reaktion kann drucklos oder unter einem Druck von 0 bis 50 bar, bevorzugt 0 bis 6 bar, durchgeführt werden.

Die Produkte werden aus der Reaktionsmischung eventuell nach Verdünnung mit Wasser abfiltriert. Wird die Säure im großem Überschuß oder ein Lösungsmittel verwendet, so kann es vorteilhaft sein abzudestillieren und das Produkt durch Extraktion zu isolieren,

Setzt man als Ausgangsprodukt bei Verfahren 1c) zur Herstellung von 3-Cyano-2,4,5-trifluorbenzoesäure den 3-Cyano-2,4,5-trifluor-benzoesäuremethylester ein, so läßt sich die Umsetzung durch folgendes Formelschema verdeutlichen:

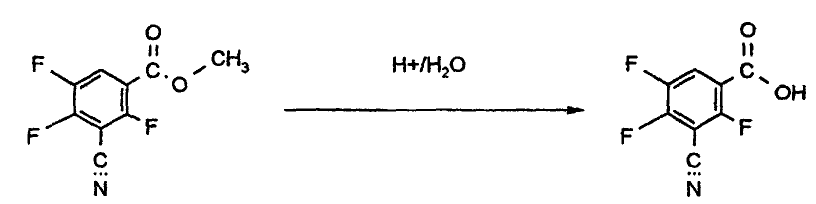

Die als Ausgangsprodukte verwendeten Ester der Formel (IV) sind neu. Ihre Herstellung ist weiter unten beschrieben.

Die Hydrolyse erfolgt in Gegenwart von Säuren und Wasser. Als Säuren können organische und anorganische starke Säuren verwendet werden. Als solche seien genannt HCl, HBr, Schwefelsäure, Methansulfonsäure, Trifluormethansulfonsäure, Benzolsulfonsäure, Toluolsulfonsäure und stark saure Ionentauscher in Gegenwart von Wasser.

Als Lösungsmittel kann die als Reagenz verwendete Säure im Überschuß oder ein organisches Lösungsmittel verwendet werden. Als organische Lösungsmittel kommen in Betracht, Säuren wie Ameisensäure, Essigsäure, Propionsäure, Ether wie Dimethoxyethan, Dioxan, Ketone wie Aceton, Butanon.

Die Reaktionskomponenten können in beliebiger Reihenfolge zugegeben werden. Anschließend wird auf die benötigte Temperatur erhitzt.

Die Reaktionstemperatur liegt im Bereich von 0 bis 200°C, bevorzugt 20 bis 150°C.

Die Reaktion kann drucklos oder unter einem Druck von 0 bis 50 bar, bevorzugt 0 bis 6 bar, durchgeführt werden.

Die Produkte werden aus der Reaktionsmischung eventuell nach Verdünnung mit Wasser extrahiert. Wird die Säure im großem Überschuß oder ein Lösungsmittel verwendet, so kann es vorteilhaft sein abzudestillieren.

Wie bereits erwähnt sind die Verbindungen der Formel (IV) neu.

Setzt man zu ihrer Herstellung gemäß Verfahren 3) als Ausgangsprodukt 2,4-Dichlor-5-fluor-isophthalodinitril ein, so läßt sich die Umsetzung durch folgendes Formelschema wiedergeben:

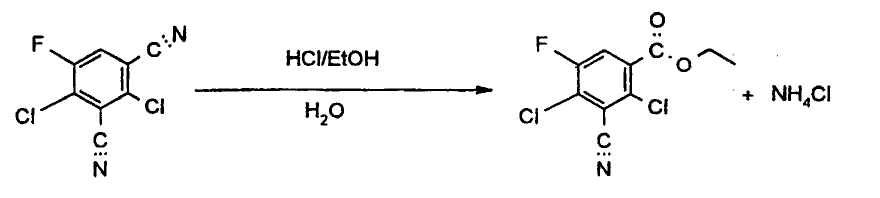

Die Reaktion verläuft intermediär über den Iminoester und dessen Hydrolyse mit Wasser. Als Nebenreaktion wird die Bildung des entsprechenden Amids beobachtet. Wird kein Wasser zugesetzt, so wird die Amidbildung zur Hauptreaktion (siehe unten).

2,4,5-Trifluor-isophthalodinitril ist literaturbekannt (EP-A-307 897). 2,4-Dichlor-5-fluor-isophthalodinitril ist neu, seine Herstellung ist weiter unten beschrieben.

Die Herstellung der Verbindung der Formel (II) erfolgt durch Hydrolyse der entsprechenden Dinitrile mit Säuren in Gegenwart von Wasser und Alkoholen.

Die Umsetzung erfolgt in Gegenwart von 1 bis 10 Äquivalenten Wasser und primären und sekundären aliphatischen Alkoholen. Bevorzugt sind Methanol, Ethanol, Propanol und Butanol. Als Säuren können organische und anorganische starke Säuren verwendet werden, wie HCI, HBr, Schwefelsäure, Methansulfonsäure, Trifluormethansulfonsäure, Benzolsulfonsäure, Toluolsulfonsäure und stark saure Ionentauscher.

Auf 1 Mol Dinitril können neben 1 bis 10 Mol Alkohol auch 1 bis 10 Mol Wasser verwendet werden.

Die Reaktion kann mit oder ohne Lösungsmittel durchgeführt werden. Als Lösungsmittel kann der als Reagenz verwendete Alkohol im Überschuß oder ein inertes organisches Lösungsmittel verwendet werden. Als inerte Lösungsmittel kommen in Betracht: Alle inerten organischen Lösungsmittel wie Kohlenwasserstoffe wie Pentan, Hexan, Heptan, Petrolether, Benzin, Ligroin, Benzol, Toluol; halogenierte Kohlenwasserstoffe wie Dichlormethan, Chloroform, Chlorbenzol, Dichlorbenzol, Trichlorethan; Ether wie Diethylether, Dipropylether, Dibutylether, Ethylenglykoldimethylether, Diethylenglykoldiethylether.

Dinitril und Alkohol werden vorgelegt und Säure zugegeben, danach wird das Wasser zugegeben. Das für die Reaktion benötigte Wasser kann aber auch direkt vorgelegt werden.

Die Reaktionstemperatur liegt im Bereich von -20 bis 150°C. Bevorzugt sind 10 bis 100°C.

Die Umsetzung kann bei Normaldruck oder erhöhtem Druck von 0 bis 50 bar durchgeführt werden. Bevorzugt sind 0 bis 6 bar.

Das Reaktionsgemisch wird mit Wasser verdünnt und extrahiert. Falls der Alkohol in großem Überschuß oder ein inertes Lösungsmittel verwendet wurden, kann das Lösungsmittel zuvor abdestilliert werden. Als Nebenprodukt der Reaktion entstandenes Amid kann abgetrennt werden.

Wie bereits erwähnt sind die Verbindungen der Formel (II) neu.

Setzt man zu ihrer Herstellung gemäß Verfahren 3) als Ausgangsprodukt 2,4,5-Trifluor-isophthalodinitril ein, so läßt sich die Umsetzung durch folgendes Formelschema wiedergeben:

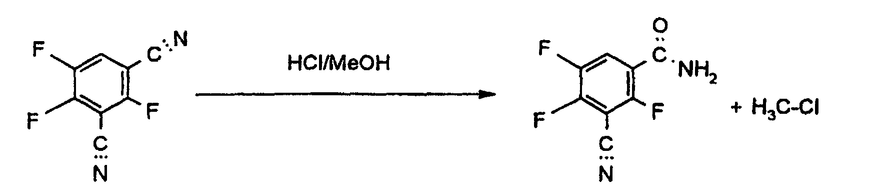

Die Reaktion läuft über die intermediär gebildeten Iminoester, aus denen durch Abspaltung des Alkylrestes die Amide gebildet werden.

2,4,5-Trifluor-isophthalodinitril ist literaturbekannt (EP-A-307 897). 2,4-Dichlor-5-fluor-isophthalodinitril ist neu, die Herstellung ist weiter unten beschrieben.

Die Umsetzung erfolgt mit primären oder sekundären aliphatischen Alkoholen in Gegenwart von Säuren. Bevorzugt sind Methanol, Ethanol, Propanol und Butanol.. Besonders bevorzugt ist Methanol. Als Säuren können organische und anorganische starke Säuren verwendet werden, wie HCl, HBr.

Auf 1 Mol Dinitril können 1 bis 10 Mol Alkohol verwendet werden.

Die Reaktion kann mit oder ohne Lösungsmittel durchgeführt werden. Als Lösungsmittel kann der als Reagenz verwendete Alkohol im Überschuß oder ein inertes organisches Lösungsmittel verwendet werden. Als inerte Lösungsmittel kommen in Betracht: Alle inerten organischen Lösungsmittel wie Kohlenwasserstoffe wie Pentan, Hexan, Heptan, Petrolether, Benzin, Ligroin, Benzol, Toluol; halogenierte Kohlenwasserstoffe wie Dichlormethan, Chloroform, Chlorbenzol, Dichlorbenzol, Trichlorethan, Ether wie Diethylether, Dipropylether, Dibutylether, Ethylenglykoldimethylether, Diethylenglykoldiethylether.

Dinitril und Alkohol werden vorgelegt und Säure zugegeben.

Die Reaktionstemperatur liegt im Bereich von -20 bis 150°C. Bevorzugt -0° bis 100°C.

Die Umsetzung kann bei Normaldruck oder erhöhtem Druck von 0 bis 50 bar durchgeführt werden. Bevorzugt sind 0 bis 6 bar.

Die Produkte werden aus der Reaktionsmischung eventuell nach Verdünnung mit Wasser abfiltriert. Falls der Alkohol in großem Überschuß oder ein inertes Lösungsmittel verwendet wurde, kann vorher abdestilliert werden.

Verbindungen der Formel (III), in denen X und Y nicht gleichzeitig für F stehen, sind neu. Ausgehend von 2,4,5-Trifluor-isophthalodinitril, läßt sich ihre Herstellung gemäß Verfahren 5) durch folgendes Formelschema wiedergeben: 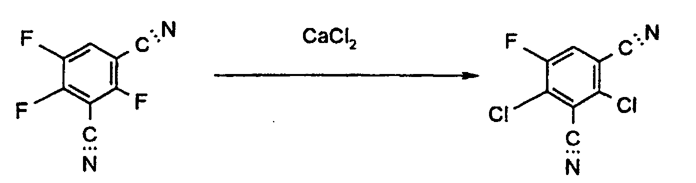 2,4,5-Trifluor-isophthalodinitril ist literaturbekannt (EP-A-307 897).

Der Halogenaustausch erfolgt durch Umsetzung mit anorganischen Chloridsalzen.

Als anorganische Chloridsalze können MgCl₂ und CaCl₂ verwendet werden. Die Reaktion kann auch katalysiert werden durch z.B. Tetraalkylammoniumsalze, Kronenether etc.

Das anorganische Salz wird mit 0,5 bis 10 Mol pro auszutauschendem Fluor eingesetzt. Bevorzugt sind 0,5 bis 2 Mol.

Die Reaktion kann mit oder ohne Lösungsmittel durchgeführt werden. Als Lösungsmittel kommen in Frage alle inerten organischen Lösungsmittel, z.B. Pentan, Hexan, Heptan, Petrolether, Benzin, Ligroin, Benzol, Toluol, Dichlormethan, Chloroform, Chlorbenzol, Dichlorbenzol, Trichlorethan, Ether wie Dibutylether, Ethylenglykoldimethylether, Diethylenglykoldiethylether, Ketone wie Aceton, Methylethylketon, Cyclohexanon, sowie N-Methylpyrrolidinon, Dimethylsulfon, Sulfolan.

Die Substanzen werden gemischt und auf die gewünschte Temperatur erhitzt. Die Reihenfolge der Zugabe spielt keine Rolle. Je nach Reaktionsführung können ein oder zwei Fluoratome gegen Chlor ausgetauscht werden.

Die Reaktionstemperatur liegt im Bereich von 50 bis 350°C. Bevorzugt sind 90 bis 250°C.

Die Reaktion kann mit oder ohne Druck durchgeführt werden. Bei Verwendung niedrig siedender Lösungsmittel kann die Durchführung unter Druck günstig sein. Druckbereich: 0 bis 100 bar Überdruck. Bevorzugt: 0 bis 50 bar.

Die Produkte werden isoliert indem man die anorganischen Salze abfiltriert und das Filtrat fraktioniert destilliert. Wurde ein mit Wasser mischbares Lösungsmittel verwendet, so kann auch auf Wasser gegossen und extrahiert werden.

3-Cyan-2,4-dihalogen-5-fluor-benzoesäure kann z.B. zur Herstellung der folgenden aus US-P-4 990 517 bekannten Verbindungen (VIII) verwendet werden:

7-Chlor-8-cyan-1-cyclopropyl-6-fluor-1,4-dihydro-4-oxo-3-chinolincarbonsäure, 7-Chlor-8-cyan-1-cyclopropyl-6-fluor-1,4-dihydro-4-oxo-3-chinolincarbonsäuremethylester, 8-Cyan-1-cyclopropyl-6,7-difluor-1,4-dihydro-4-oxo-3-chinolincarbonsäure, 8-Cyan-1-cyclopropyl-6,7-difluor-1,4-dihydro-4-oxo-3-chinolincarbonsäureethylester.

Dazu wird z.B. 3-Cyano-2,4,5-trifluorbenzoesäure in Form ihres Säurechlorids mit ß-Dimethylamino-acrylsäureester der Formel (V) umgesetzt und das erhaltene Produkt der Formel (VI) mit Cyclopropylamin zu einer Verbindung der Formel (VII) weiter umgesetzt und anschließend die obengenannnte Verbindung (VIII) erhalten:

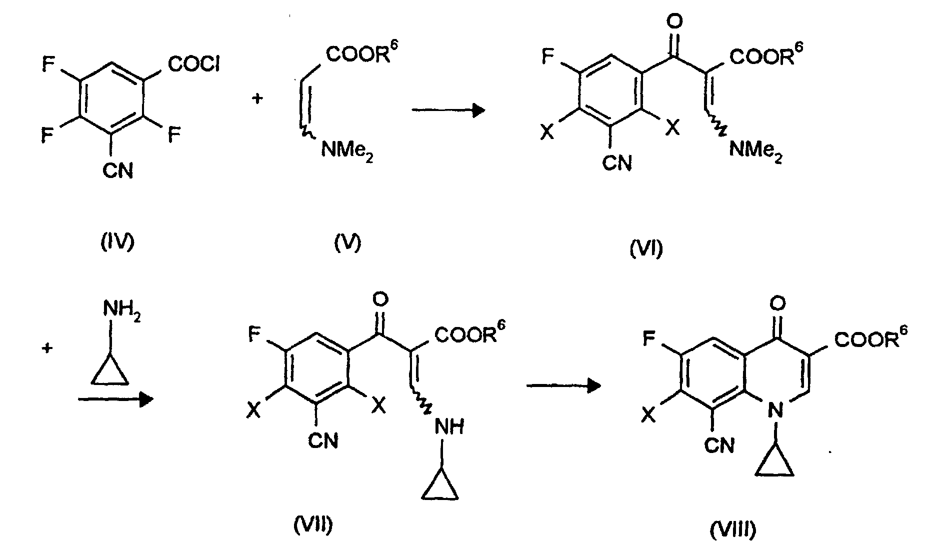

In obigem Formelschema steht
- X: für Halogen, insbesondere Fluor oder Chlor,
- R⁶: für C₁₋₄-Alkyl, insbesondere Methyl oder Ethyl.

Ebenso ist es möglich, eine Verbindung der Formel (IV) direkt mit β-Cyclopropylamino-acrylsäureester umzusetzen: 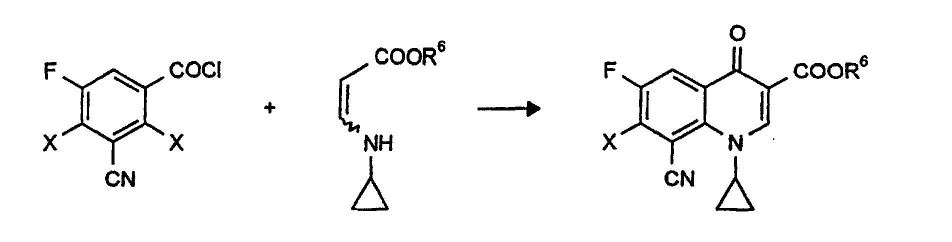 (In den Formeln haben X und R⁶ die oben angegebene Bedeutung.)

Das Säurechlorid von 3-Cyan-2,4,5-trifluorbenzoesäure kann aus den Estern der Formel (IV) nach folgendem Schema hergestellt werden:

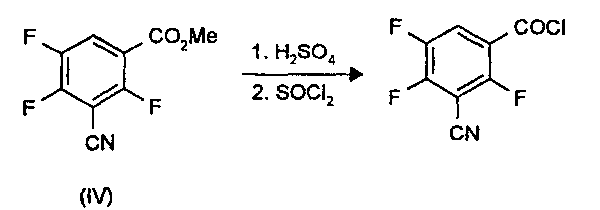

Aus den Verbindungen der Formel (VIII) können durch Umsetzung mit geeigneten Aminen antibakteriell wirksame Verbindungen hergestellt werden.

Setzt man z.B. 7-Chlor-8-cyan-1-cyclopropyl-6-fluor-1,4-dihydro-4-oxo-3-chinolincarbonsäure und 2,8-Diazabicyclo[4.3.0]nonan um, so kann der Reaktionsverlauf durch das folgende Formelschema wiedergegeben werden:

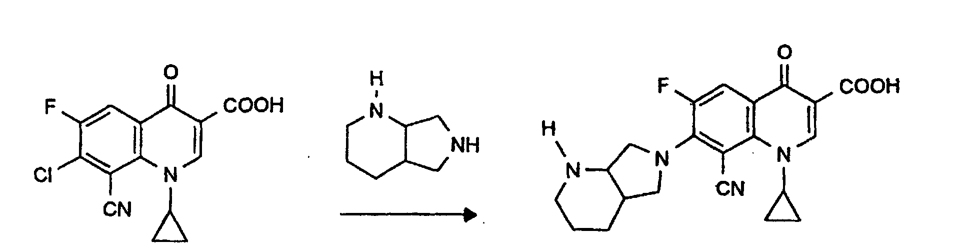

Die Herstellung dieser Verbindungen ist in der nicht vorveröffentlichten DE-A-196 33 805 der Anmelderin beschrieben.

Die folgenden Beispiele zeigen die vorliegende Erfindung ohne ihren Umfang zu beschränken.

### Beispiel 1 (Verfahren 1a)

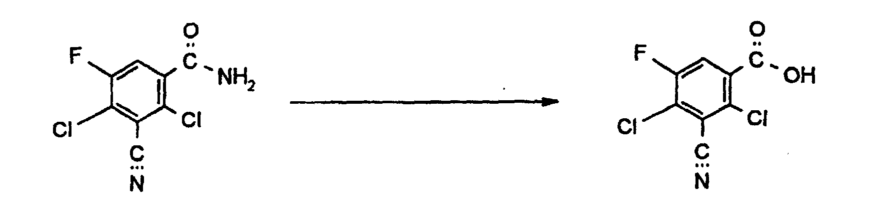

| Menge | Mol | Text |
|---|---|---|
| 0,4 g | 1,72 m | 3-Cyano-2,4-dichlor-5-fluor-benzamid und |
| 5 ml | | konzentrierte Salzsäure wurden drei Stunden unter Rückfluß erhitzt. Danach wurde eingedampft und der Rückstand im Exsikkator über Schwefelsäure getrocknet. Ausbeute: 370 mg Reinheit: 84 % (HPLC-Fläche) 3-Cyano-2,4-dichlor-5-fluor-benzoesäure 12 % (HPLC-Fläche) 3-Cyano-2,4-dichlor-5-fluorbenzamid (Ausgangsmaterial) |

### Beispiel 2 (Verfahren 1b)

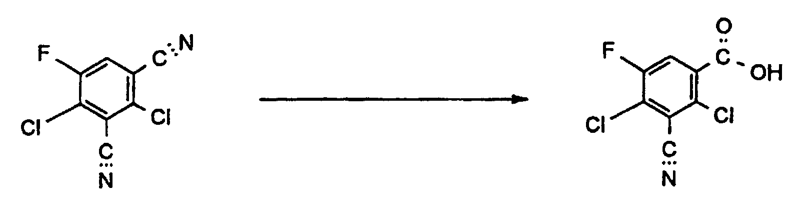

| Menge | Mol | |
|---|---|---|
| 2,5 g | 12 m | 2,4-Dichlor-5-fluor-isophthalodinitril wurden in |
| 25 ml | | Methanol suspendiert, auf 0° gekühlt und mit gasförmiger Salzsäure gesättigt. Der Ansatz blieb 60 Stunden bei -16°C stehen. Danach wurde eingedampft. Der Rückstand wurde mit |
| 38 ml | | konzentrierter Salzsäure versetzt und drei Stunden zum Rückfluß erhitzt. Anschließend wurde auf |
| 120 ml | | Wasser gegossen, der Niederschlag abgesaugt und im Exsikkator über Schwefelsäure getrocknet. Ausbeute: 1,86 g Reinheit: 87 % 3-Cyano-2,4-dichlor-5-fluor-benzoesäure 10 % 3-Cyano-2,4-dichlor-5-fluor-benzamid |

### Beispiel 3 (Verfahren 1b)

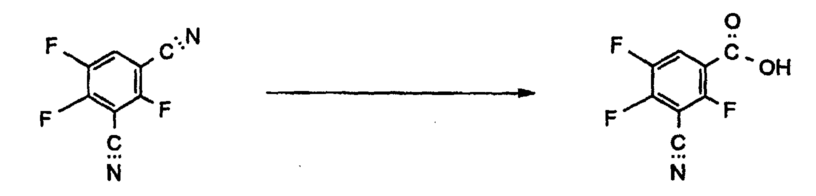

| Menge | Mol | Text |
|---|---|---|
| 1 g | 5,5 m | 2,4,5-Trifluor-isophthalodinitril, |
| 6,3 ml | | Wasser, |
| 6,3 ml | | Eisessig und |
| 0,63 ml | | 96 %ige Schwefelsäure wurden 24 Stunden zum Rückfluß erhitzt. Danach wurde auf |
| 50 ml | | Wasser gegossen und mit |
| 25 ml | | Dichlormethan versetzt. Die wässrige Phase wurde nun mit 45 %iger NaOH auf pH 9 gestellt, die organische Phase dann abgetrennt und zweimal mit Dichlormethan nachextrahiert. Die Extrakte wurden verworfen. Die verbliebene wässrige Phase wurde nun mit konzentrierter Salzsäure auf pH 2 gestellt und einsgesamt dreimal mit je |
| 25 ml | | Dichlormethan extrahiert. Die vereinigten Extrakte wurden mit Natriumsulfat getrocknet und eingedampft. Rückstand: 470 mg Reinheit: 80 % (GC/MS-Fläche) |

### Beispiel 4 (Verfahren 1c)

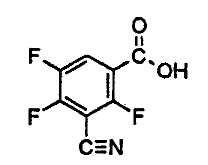

| Menge | Mol | Text |
|---|---|---|
| 2 g | 8,7 m | 3-Cyano-2,4,5-trifluor-benzoesäure-ethylester (Reinheit 79 %), |
| 10 ml | | Eisessig |
| 10 ml | | Wasser und |
| 1 ml | | 96 %ige Schwefelsäure werden 7,5 Stunden zum Rückfluß erhitzt. Danach wird abgekühlt, auf |
| 100 ml | | Wasser gegossen und dreimal mit Dichlormethan extrahiert. Der Extrakt wird mit |
| 100 ml | | Wasser versetzt und unter Rühren der pH der wässrigen Phase auf 8,5 gestellt. Die organische Phase wird abgetrennt, die wässrige Phase mit Dichlormethan nachextrahiert. Die organischen Extrakte werden verworfen. Zur wässrigen Phase gibt man nun |
| 50 ml | | Dichlormethan und säuert mit Schwefelsäure an. Das Dichlormethan wird abgetrennt und nochmals mit Dichlormethan nachextrahiert. Die vereinigten Extrakte dann mit Na₂SO₄ getrocknet und eingedampft. Der Rückstand wird im Exsikkator über KOH getrocknet. Ausbeute: 1,2 g (81 % der Theorie) Reinheit: 95 % (HPLC-Fläche) Schmelzpunkt: 146°C |

### Beispiel 5 (Verfahren 3)

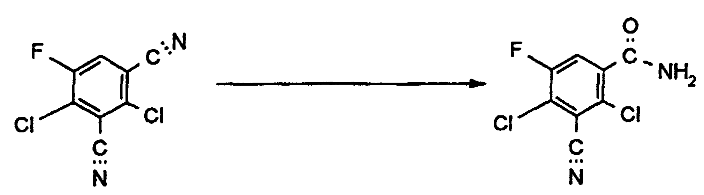

| Menge | Mol | Text |
|---|---|---|
| 9 g | 42 m | 2,4-Dichlor-5-fluor-isophthalodinitril wurden in |
| 90 ml | | Methanol suspendiert, auf 5° gekühlt und bis zur Sättigung gasförmige Salzsäure eingeleitet. Es entstand eine Lösung, die 24 Stunden bei Raumtemperatur gerührt wurde. Danach wurde eingedampft, der Rückstand mit Dichlormethan verrührt und abgesaugt. Ausbeute: 8,04 g Reinheit: 95 % (HPLC-Fläche) Schmelzpunkt: 178° |

### Beispiel 6 (Verfahren 3)

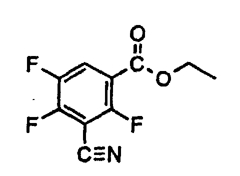

| Menge | Mol | Text |
|---|---|---|
| 5,52 g | 30 m | 2,4,5-Trifluor-isophthalsäuredinitril (Reinheit 86 %) werden in |
| 60 ml | | trockenem Ethanol vorgelegt und unter Eiskühlung gasförmige Salzsäure bis zur Sättigung eingeleitet. Es wird vier Stunden bei Raumtemperatur nachgerührt, dann |
| 4,8 ml | 0,266 | Wasser zugegeben und vier Stunden zum Rückfluß erhitzt. Danach wird eingedampft, der Rückstand zwischen Wasser und Chloroform verteilt. Die organische Phase wird abgetrennt, die wässrige Phase noch zweimal mit Chloroform nachextrahiert. Die gesammelten Extrakte werden mit Na₂SO₄ getrocknet und eingedampft. Der Rückstand wird im Kugelrohr destilliert. Siedepunkt: 220° (42 mbar) Ausbeute: 4,21 g (55 % der Theorie) Reinheit: 79 % (HPLC-Fläche) ¹H-NMR (CDCl₃): 8,1 ppm (m, 1H, Ar-H) 4,4 ppm (q, J=8 Hz, 2H, -OCH₂-) 1,4 ppm (t, J=8 Hz, 3H, -CH₃) |

### Beispiel 7 (Verfahren 3)

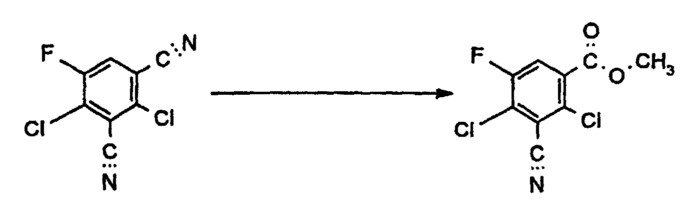

| Menge | Mol | Text |
|---|---|---|
| 0,5 g | 2,3 m | 2,4-Dichlor-5-fluor-isophtholodinitril wurden in |
| 10 ml | | Methanol gelöst und unter guter Kühlung bei 0° mit gasförmiger HCl gesättigt. Anschließend wurde der Ansatz 72 Stunden bei -10° stehen lassen. Danach wurde |
| 1 ml | | 96 %iges wässriges Methanol zugegeben und drei Stunden unter Rückfluß erhitzt. Anschließend wurde im Vakuum eingedampft, der Rückstand zwischen Chloroform und gesättigter Bicarbonatlösung verteilt, die organische Phase abgetrennt, mit Natriumsulfat getrocknet und eingedampft. Rückstand: 410 mg Nach HPLC sind darin 10 % Ausgangsmaterial 7,5 % Amid 76 % Methylester enthalten. |

### Beispiel 8 (Verfahren 5)

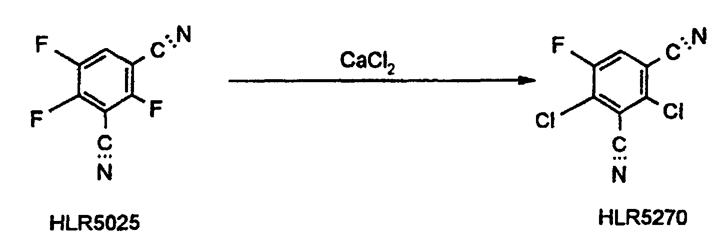

| Menge | Mol | Text |
|---|---|---|
| 13,5 g | 74 m | 2,4,5-Trifluor-isophthalodinitril, |
| 125 ml | | Sutfolan and |
| 17,7 g | | frisch gepulvertes Calciumchlorid werden 24 Stunden auf 200° erhitzt. anschließend wird auf |
| 1200 ml | | Wasser gegossen. Der feine Niederschlag wird abgesaugt und getrocknet. Ausbeute 13,8 g Zur Reinigung kann über Kieselgel mit Toluol/Hexan filtriert werden. Ausbeute:12,8 g Reinheit: 96 % HPLC-Fläche Schmelzpunkt: 119° |

### Beispiel 9 (Verfahren 5)

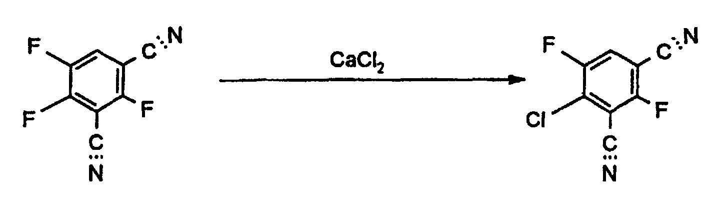

| Menge | Mol | Text |
|---|---|---|
| 0,55 g | 3 m | 2,4,5-Trifluor-isophthalodinitril, |
| 5 ml | | Sulfolan und |
| 0,37 g | 3,3 m | frisch gepulvertes Calciumchlorid wurden 1,5 Stunden auf 200° erhitzt. anschließend wurde auf |
| 100 ml | | Wasser gegossen und zweimal mit Ether extrahiert. Der Extrakt wurde mit Natriumsulfat getrocknet und eingedampft. Ausbeute: 0,56 g Zusammensetzung: 75 % 4-Chlor-2,5-difluor-isophthalodinitril 25 % 5-Fluor-2,4-dichlor-isophthalodinitril (HPLC-fläche) |

## Patentansprüche

1. Verfahren zur Herstellung von 3-Cyano-2,4-dihalogen-5-fluorbenzoesäuren der Formel (I) 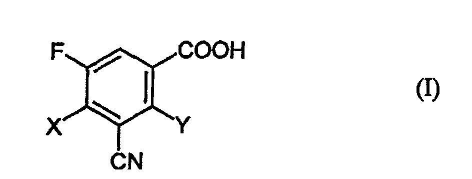 in welcher
X und Y unabhängig voneinander für Halogen stehen,
durch hydrolytische Spaltung von
a) 3 -Cyano-2,4-dihalogen-5-fluorbenzoesäureamiden der Formel (II) 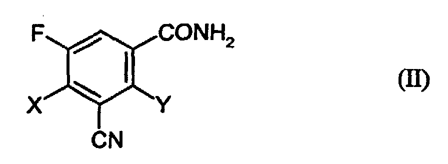 in welcher
X und Y unabhängig voneinander für Halogen stehen,
oder
b) 1,3-Dicyano-2,4-dihalogen-5-fluorbenzolen der Formel (III) 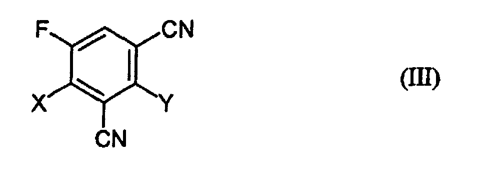 in welcher
X und Y unabhängig voneinander für Halogen stehen,
oder
c) 3-Cyano-2,4-dihalogen-5-fluorbenzoesäureestern der Formel (IV), ausgenommen 3-Cyano-2,4,5-trifluorbenzoesäuremethylester, 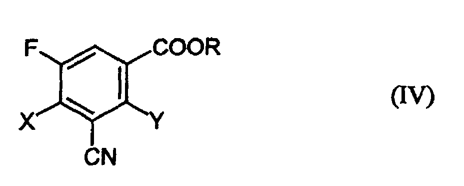 in welcher
X und Y unabhängig voneinander für Halogen stehen und
R für C₁₋₄-Alkyl steht, das gegebenenfalls substituiert sein kann.

2. Verbindungen der Formeln (II) 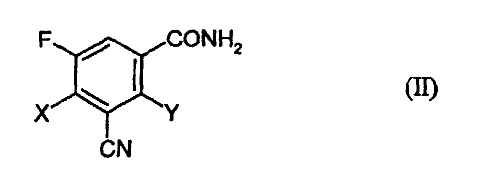 und (IV) 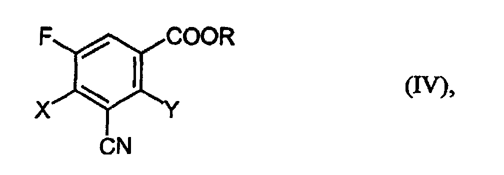 in welcher
X und Y unabhängig voneinander für Halogen stehen und
R für C₁₋₄-Alkyl steht, das gegebenenfalls substituiert ist,
ausgenommen 3-Cyano-2,4,5-trifluorbenzoesäuremethylester.

3. Verfahren zur Herstellung der 3-Cyano-2,4-dihalogen-5-fluor-benzoesäureamide der Formel (II) 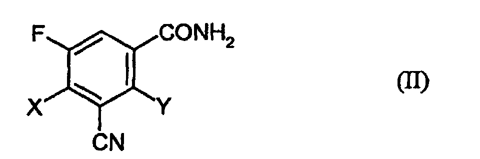 bzw. der Ester der Formel (IV) 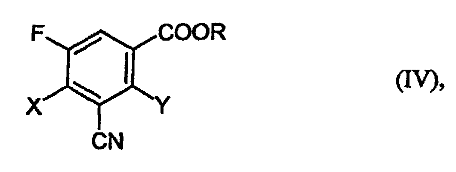 in welcher
X und Y unabhängig voneinander für Halogen stehen und
R für C₁₋₄-Alkyl steht, das gegebenenfalls substituiert ist,
das **dadurch gekennzeichnet ist, daß** man 1,3-Dicyano-2,4-dihalogen-5-fluorbenzole der Formel (III) 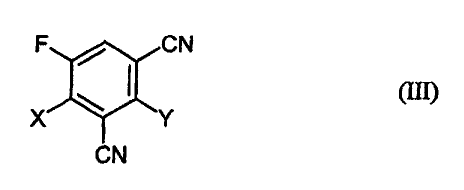 in welcher
X und Y die oben angegebene Bedeutung haben,
in Gegenwart von Wasser oder in Gegenwart von Alkoholen hydrolysiert.

4. Die neuen Verbindungen der Formel (III) 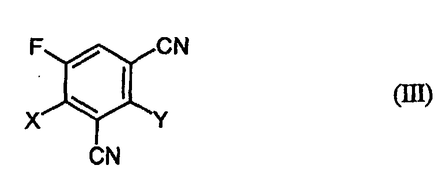 in welcher
X und Y für ungleiche Reste aus der Gruppe Fluor oder Chlor stehen oder beide Reste für Chlor stehen.

5. Verfahren zur Herstellung der Verbindungen der Formel (III) 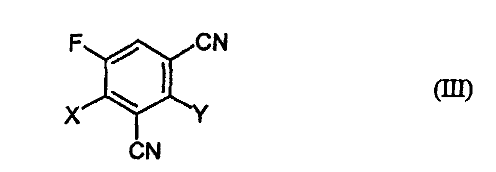 in welcher
X und Y für ungleiche Reste aus der Gruppe Fluor oder Chlor stehen oder beide Reste für Chlor stehen,
das **dadurch gekennzeichnet ist, daß** man 1,2,4-Trifluor-3,5-dicyanbenzol (2,4,5-Trifluor-isophthalodinitril) mit einem Metallchlorid umsetzt.

## Claims

1. Process for preparing 3-cyano-2,4-dihalogeno-5-fluorobenzoic acids of the formula (I) 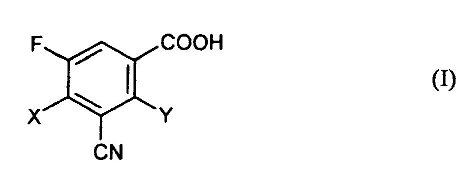 in which
X and Y independently of one another each represent halogen
by hydrolytic cleavage of
a) 3-cyano-2,4-dihalogeno-5-fluorobenzamides of the formula (II) 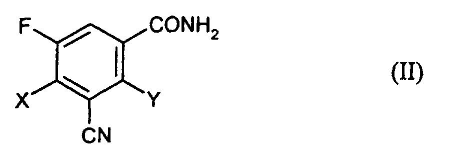 in which
X and Y independently of one another each represent halogen,
or
b) 1,3-dicyano-2,4-dihalogeno-5-fluorobenzenes of the formula (III) 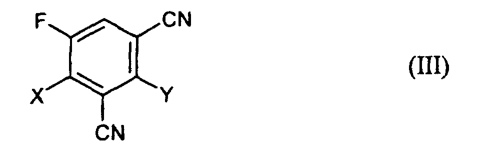 in which
X and Y independently of one another each represent halogen,
or
c) 3-cyano-2,4-dihalogeno-5-fluorobenzoic esters of the formula (IV), except for methyl 3-cyano-2,4,5-trifluorobenzoate, 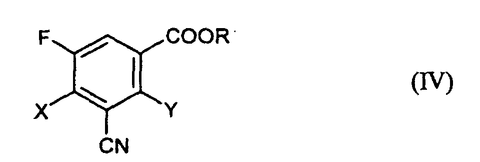 in which
X and Y independently of one another each represent halogen and
R represents C₁₋₄-alkyl which may optionally be substituted.

2. Compounds of the formulae (II) 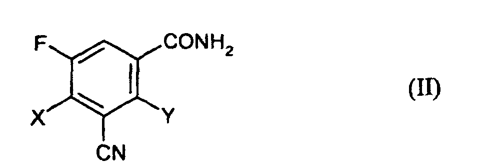 and (IV) 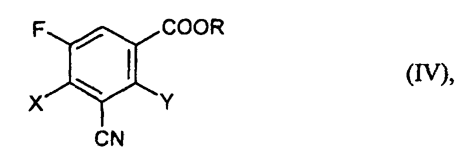 in which
X and Y independently of one another each represent halogen and
R represents C₁₋₄-alkyl which is optionally substituted,
except for methyl 3-cyano-2,4,5-trifluorobenzoate.

3. Process for preparing 3-cyano-2,4-dihalogeno-5-fluoro-benzamides of the formula (II) 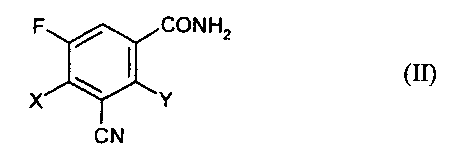 or esters of the formula (IV) 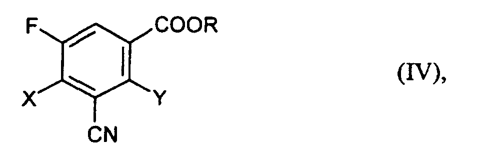 in which
X and Y independently of one another each represent halogen and
R represents C₁₋₄-alkyl which is optionally substituted,
**characterized in that** 1,3-dicyano-2,4-dihalogeno-5-fluorobenzenes of the formula (III) 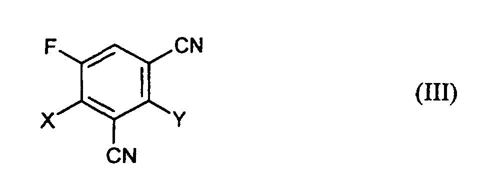 in which
X and Y are each as defined above,
are hydrolyzed in the presence of water or in the presence of alcohols.

4. The novel compounds of the formula (III) 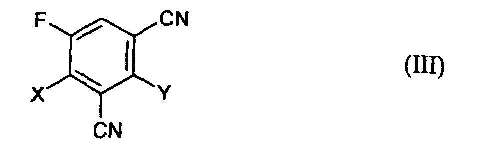 in which
X and Y represent different radicals from the group consisting of fluorine and chlorine, or both radicals represent chlorine.

5. Process for preparing the compounds of the formula (III) 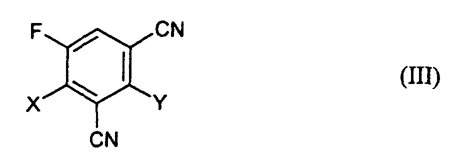 in which
X and Y represent different radicals from the group consisting of fluorine and chlorine or both radicals represent chlorine,
**characterized in that** 1,2,4-trifluoro-3,5-dicyanobenzene (2,4,5-trifluoroisophthalonitrile) is reacted with a metal halide.

## Revendications

1. Procédé de production d'acides 3-cyano-2,4-dihalogéno-5-fluorobenzoïques de formule (I) 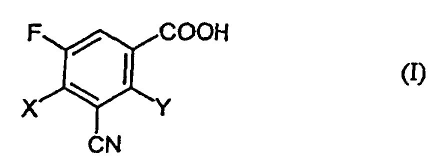 dans laquelle
X et Y, indépendamment l'un de l'autre, représentent un halogène,
par clivage hydrolytique
a) d'amides d'acides 3-cyano-2,4-dihalogéno-5-fluorobenzoïques de formule (II) 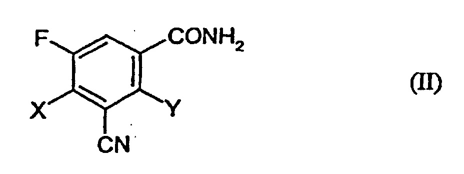 dans laquelle
X et Y, indépendamment l'un de l'autre, représentent un halogène,
ou
b) de 1,3-dicyano-2,4-dihalogéno-5-fluorobenzènes de formule (III) 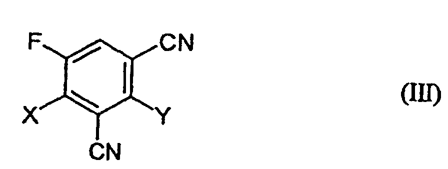 dans laquelle
X et Y, indépendamment l'un de l'autre, représentent un halogène,
ou bien
c) d'esters d'acides 3-cyano-2,4-dihalogéno-5-fluorobenzoïques de formule (IV), excepté l'ester méthylique d'acide 3-cyano-2,4,5-trifluorobenzoïque, 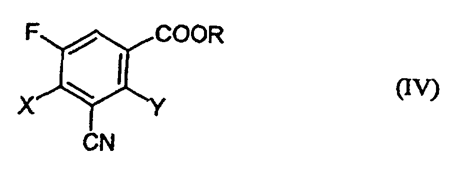 formule dans laquelle
X et Y, indépendamment l'un de l'autre, représentent un halogène et
R est un reste alkyle en C₁ à C₄ qui peut éventuellement être substitué.

2. Composés de formules (II) 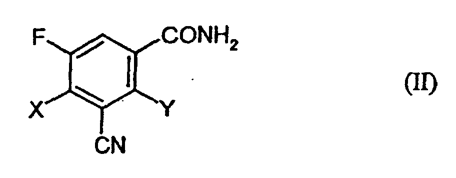 et (IV) 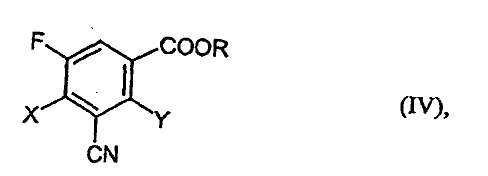 formules dans lesquelles
X et Y, indépendamment l'un de l'autre, représentent un halogène et
R est un reste alkyle en C₁ à C₄ qui est éventuellement substitué,
excepté l'ester méthylique d'acide 3-cyano-2,4,5-trifluorobenzoïque.

3. Procédé de production des amides d'acides 3-cyano-2,4-dihalogéno-5-fluorobenzoïques de formule (II) 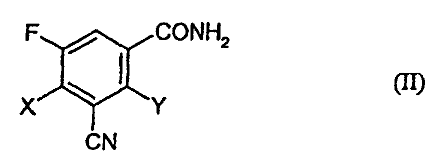 ou des esters de formule (IV) 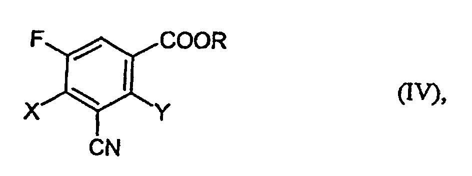 formules dans lesquelles
X et Y, indépendamment l'un de l'autre, représentent un halogène et
R est un reste alkyle en C₁ à C₄ qui est éventuellement substitué,
**caractérisé en ce qu'**on hydrolyse des 1,3-dicyano-2,4-dihalogéno-5-fluorobenzènes de formule (III) 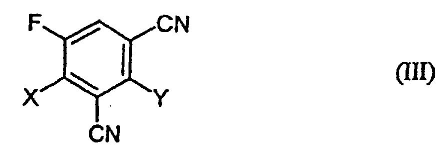 dans laquelle
X et Y ont la définition indiquée ci-dessus,
en présence d'eau ou en présence d'alcools.

4. Les composés nouveaux de formule (III) 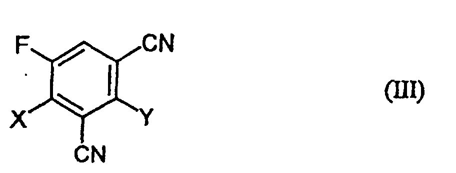 dans laquelle
X et Y sont des restes différents choisis dans le groupe du fluor ou du chlore ou bien les deux restes représentent du chlore.

5. Procédé de production des composés de formule (III) 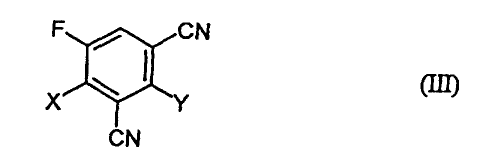 dans laquelle
X et Y sont des restes différents choisis dans le groupe du fluor ou du chlore ou bien les deux restes représentent du chlore,
**caractérisé en ce qu'**on fait réagir le 1,2,4-trifluoro-3,5-dicyanobenzène (2,4,5-trifluoro-isophtalodinitrile) avec un chlorure métallique.
